# EUROPEAN PATENT APPLICATION

(11) **EP 4 755 366 A1**
(43) Date of publication of application: **10.06.2026**
(21) Application number: 24849259.7
(22) Date of filing: 01.08.2024
(51) Int. Cl.: A61K 8/37, A61K 8/04, A61K 9/10, A61K 47/14, A61K 47/26, A61Q 1/12, A61Q 19/00

(54) **SOLID PARTICLE DISPERSION PREPARATION**

(30) Priority: 01.08.2023 JP 2023125524
(71) Applicant: Novigo Pharma, Inc., Fukuoka-shi, Fukuoka 819-0388 (JP)
(72) Inventor: ISHIHAMA Kohei, Fukuoka-shi, Fukuoka 819-0388 (JP); KITAOKA Momoko, Fukuoka-shi, Fukuoka 819-0388 (JP)
(74) Representative: Hoffmann Eitle
(86) International application number: PCT/JP2024/027553
(87) International publication number: WO 2025/028614

(57) **Abstract**

The present disclosure provides a solid particle dispersion formulation. According to the present disclosure, the solid particle dispersion formulation comprises a solid particle containing specific emulsifiers and combinations of specific emulsifiers and a water-soluble molecule, and an oily base, wherein the solid particles are dispersed in the oily base.

## Description

### [Technical Field]

The present disclosure relates to a solid particle dispersion formulation.

### [Background Art]

A stratum corneum exists in skin tissue, providing a physical barrier that non-specifically prevents invasion by invaders such as bacteria and viruses. Due to the presence of said physical barrier, the stratum corneum has poor permeability for substances, becoming an obstacle for transdermally administering drugs. In particular, hydrophilic molecules with a molecular weight exceeding 800 are not suitable for transdermal administration.

In order to allow hydrophilic compounds to be transdermally absorbed, formulation technologies have been developed in which a solid complex formed by coating a hydrophilic compound with a surfactant is dispersed in an oil phase (Patent Documents 1 and 2).

### [Prior Art Documents]

### [Patent Documents]

[Patent Document 1] WO 2006/025583
[Patent Document 2] WO 2022/196748

### [Summary of the Invention]

The present disclosure provides a solid particle dispersion formulation.

According to the present disclosure, the utility and efficacy of a dispersion formulation in which particles of a solid complex composed of two specific types of emulsifiers and a target molecule are dispersed have been clarified.

According to the present disclosure, the following inventions are provided.
(1) A dispersion formulation comprising solid particles containing a complex of a water-soluble molecule and an emulsifier, and an oily base, wherein the emulsifier comprises a glyceryl fatty acid, and said solid particles are dispersed in said oily base.
(2) The dispersion formulation according to (1), wherein the glyceryl fatty acid is selected from the group consisting of polyglyceryl polyfatty acid, polyglyceryl monofatty acid, monoglyceryl polyfatty acid, and monoglyceryl monofatty acid.
(3) The dispersion formulation according to (1) or (2), wherein the glyceryl fatty acid comprises a polyglyceryl having a number average degree of polymerization of 2 to 6.
(4) The dispersion formulation according to any one of (1) to (3), wherein the emulsifier further comprises a nonionic surfactant (e.g., a sucrose fatty acid ester).
(5) The dispersion formulation according to any one of (1) to (4), wherein the sucrose fatty acid ester is a sucrose fatty acid ester.
(6) The dispersion formulation according to any one of (1) to (5), wherein the sucrose fatty acid ester is sucrose erucate ester.
(7) The dispersion formulation according to any one of (4) to (6), wherein the emulsifier comprises 10 to 40 mol% of the sucrose fatty acid ester.
(8) The dispersion formulation according to any one of (1) to (7), wherein the Z-average size of the particles is 500 nm or less.
(9) A cosmetic comprising the dispersion formulation according to any one of (1) to (7). (21) The dispersion formulation according to any one of (1) to (7), wherein the glyceryl fatty acid comprises one or more selected from the group consisting of Glyceryl Fatty Acid Group A: polyglyceryl decaoleate, polyglyceryl polyricinoleate, polyglyceryl diisostearate, polyglyceryl tetraisostearate, glyceryl monooleate, polyoxyethylene hydrogenated castor oil, polyglyceryl pentaoleate, polyglyceryl decaisostearate, and glyceryl monocaprate.
(22) The dispersion formulation according to any one of (1) to (8), wherein the glyceryl fatty acid comprises one or more selected from the group consisting of Glyceryl Fatty Acid Group B: polyglyceryl-10 decaoleate, polyglyceryl-6 polyricinoleate, polyglyceryl-6 polyricinoleate, polyglyceryl-2 diisostearate, polyglyceryl-2 tetraisostearate, glyceryl monooleate, polyoxyethylene hydrogenated castor oil, polyglyceryl-10 pentaoleate, polyglyceryl-10 decaisostearate, and glyceryl monocaprate.
(31) The dispersion formulation according to any one of (1) to (8), wherein the glyceryl fatty acid comprises polyglyceryl decaoleate.
(32) The dispersion formulation according to any one of (1) to (8), wherein the glyceryl fatty acid comprises polyglyceryl polyricinoleate.
(33) The dispersion formulation according to any one of (1) to (8), wherein the glyceryl fatty acid comprises polyglyceryl diisostearate.
(34) The dispersion formulation according to any one of (1) to (8), wherein the glyceryl fatty acid comprises polyglyceryl tetraisostearate.
(35) The dispersion formulation according to any one of (1) to (8), wherein the glyceryl fatty acid comprises glyceryl monooleate.
(36) The dispersion formulation according to any one of (1) to (8), wherein the glyceryl fatty acid comprises polyoxyethylene hydrogenated castor oil.
(37) The dispersion formulation according to any one of (1) to (8), wherein the glyceryl fatty acid comprises polyglyceryl pentaoleate.
(38) The dispersion formulation according to any one of (1) to (8), wherein the glyceryl fatty acid comprises polyglyceryl decaisostearate.
(39) The dispersion formulation according to any one of (1) to (8), wherein the glyceryl fatty acid comprises glyceryl monocaprate.
(41) The dispersion formulation according to (4), wherein the glyceryl fatty acid comprises polyglyceryl decaoleate.
(42) The dispersion formulation according to (4), wherein the glyceryl fatty acid comprises polyglyceryl polyricinoleate.
(43) The dispersion formulation according to (4), wherein the glyceryl fatty acid comprises polyglyceryl diisostearate.
(44) The dispersion formulation according to (4), wherein the glyceryl fatty acid comprises polyglyceryl tetraisostearate.
(45) The dispersion formulation according to (4), wherein the glyceryl fatty acid comprises glyceryl monooleate.
(46) The dispersion formulation according to (4), wherein the glyceryl fatty acid comprises polyoxyethylene hydrogenated castor oil.
(47) The dispersion formulation according to (4), wherein the glyceryl fatty acid comprises polyglyceryl pentaoleate.
(48) The dispersion formulation according to (4), wherein the glyceryl fatty acid comprises polyglyceryl decaisostearate.
(49) The dispersion formulation according to (4), wherein the glyceryl fatty acid comprises glyceryl monocaprate.
(51) The dispersion formulation according to (5), wherein the glyceryl fatty acid comprises polyglyceryl decaoleate.
(52) The dispersion formulation according to (5), wherein the glyceryl fatty acid comprises polyglyceryl polyricinoleate.
(53) The dispersion formulation according to (5), wherein the glyceryl fatty acid comprises polyglyceryl diisostearate.
(54) The dispersion formulation according to (5), wherein the glyceryl fatty acid comprises polyglyceryl tetraisostearate.
(55) The dispersion formulation according to (5), wherein the glyceryl fatty acid comprises glyceryl monooleate.
(56) The dispersion formulation according to (5), wherein the glyceryl fatty acid comprises polyoxyethylene hydrogenated castor oil.
(57) The dispersion formulation according to (5), wherein the glyceryl fatty acid comprises polyglyceryl pentaoleate.
(58) The dispersion formulation according to (5), wherein the glyceryl fatty acid comprises polyglyceryl decaisostearate.
(59) The dispersion formulation according to (5), wherein the glyceryl fatty acid comprises glyceryl monocaprate.
(71) The dispersion formulation according to (6), wherein the glyceryl fatty acid comprises polyglyceryl decaoleate.
(72) The dispersion formulation according to (6), wherein the glyceryl fatty acid comprises polyglyceryl polyricinoleate.
(73) The dispersion formulation according to (6), wherein the glyceryl fatty acid comprises polyglyceryl diisostearate.
(74) The dispersion formulation according to (6), wherein the glyceryl fatty acid comprises polyglyceryl tetraisostearate.
(75) The dispersion formulation according to (6), wherein the glyceryl fatty acid comprises glyceryl monooleate.
(76) The dispersion formulation according to (6), wherein the glyceryl fatty acid comprises polyoxyethylene hydrogenated castor oil.
(77) The dispersion formulation according to (6), wherein the glyceryl fatty acid comprises polyglyceryl pentaoleate.
(78) The dispersion formulation according to (6), wherein the glyceryl fatty acid comprises polyglyceryl decaisostearate.
(79) The dispersion formulation according to (6), wherein the glyceryl fatty acid comprises glyceryl monocaprate.
(81) The dispersion formulation according to (7), wherein the glyceryl fatty acid comprises polyglyceryl decaoleate.
(82) The dispersion formulation according to (7), wherein the glyceryl fatty acid comprises polyglyceryl polyricinoleate.
(83) The dispersion formulation according to (7), wherein the glyceryl fatty acid comprises polyglyceryl diisostearate.
(84) The dispersion formulation according to (7), wherein the glyceryl fatty acid comprises polyglyceryl tetraisostearate.
(85) The dispersion formulation according to (7), wherein the glyceryl fatty acid comprises glyceryl monooleate.
(86) The dispersion formulation according to (7), wherein the glyceryl fatty acid comprises polyoxyethylene hydrogenated castor oil.
(87) The dispersion formulation according to (7), wherein the glyceryl fatty acid comprises polyglyceryl pentaoleate.
(88) The dispersion formulation according to (7), wherein the glyceryl fatty acid comprises polyglyceryl decaisostearate.
(89) The dispersion formulation according to (7), wherein the glyceryl fatty acid comprises glyceryl monocaprate.
(101) A dispersion formulation comprising solid particles containing a water-soluble molecule and one or more types of emulsifiers, and an aqueous base, wherein said solid particles are dispersed in said aqueous base.
(102) The dispersion formulation according to (101), wherein said one or more types of emulsifiers comprise a sucrose fatty acid ester.
(103) The dispersion formulation according to (101) or (102), wherein said one or more types of emulsifiers comprise sucrose erucate ester.
(104) The dispersion formulation according to any one of (101) to (103), wherein the Z-average size of the particles is 300 nm or less, or 200 nm or less.
(105) The dispersion formulation according to any one of (101) to (104), wherein the Z-average size of the particles is 50 nm or more.
(106) The dispersion formulation according to any one of (101) to (105), wherein the water-soluble molecule comprises nicotinamide mononucleotide.
(107) The dispersion formulation according to any one of the above, which exhibits dispersibility over 1 month or more, preferably 2 months or more, under a temperature condition of 25°C.
(108) The dispersion formulation according to (107), wherein the emulsifier comprises a nonionic surfactant and a polyglyceryl fatty acid.
(109) The dispersion formulation according to (108), wherein the nonionic surfactant comprises a sucrose fatty acid ester.
(110) The dispersion formulation according to (107), wherein the glycerin polymerization number of the polyglyceryl fatty acid is any of 2 to 6.
(111) The dispersion formulation according to (108), wherein the glycerin polymerization number of the polyglyceryl fatty acid is any of 2 to 6.
(112) The dispersion formulation according to (110), wherein the polyglyceryl fatty acid is one or more selected from the group consisting of polyglyceryl-6 polyricinoleate, polyglyceryl-6 polyricinoleate, polyglyceryl-10 decaoleate, polyglyceryl-10 pentaoleate, polyglyceryl-2 diisostearate, and polyglyceryl-10 decaisostearate.
(113) The dispersion formulation according to (111), wherein the polyglyceryl fatty acid is one or more selected from the group consisting of polyglyceryl-6 polyricinoleate, polyglyceryl-6 polyricinoleate, polyglyceryl-10 decaoleate, polyglyceryl-10 pentaoleate, polyglyceryl-2 diisostearate, and polyglyceryl-10 decaisostearate.
(114) The dispersion formulation according to (109), wherein the sucrose fatty acid ester comprises sucrose erucate ester.
(115) The dispersion formulation according to (111), wherein the sucrose fatty acid ester comprises sucrose erucate ester.
(116) The dispersion formulation according to (112), wherein the sucrose fatty acid ester comprises sucrose erucate ester.
(117) The dispersion formulation according to (113), wherein the sucrose fatty acid ester comprises sucrose erucate ester.
(131) A dispersion formulation comprising solid particles containing a water-soluble molecule and one or more (preferably two or more) types of emulsifiers, and an aqueous base, wherein said solid particles are dispersed in said aqueous base.
(132) The dispersion formulation according to (131), wherein said one or more types of emulsifiers comprise a sucrose fatty acid ester.
(133) The dispersion formulation according to (131) or (132), wherein said one or more types of emulsifiers comprise sucrose erucate ester.
(134) The dispersion formulation according to any one of (131) to (133), wherein the Z-average size of the particles is 200 nm or less.
(135) The dispersion formulation according to any one of (131) to (134), wherein the emulsifier comprises two or more types of emulsifiers.
(136) The dispersion formulation according to any one of (131) to (135), wherein the emulsifier comprises a polyglyceryl fatty acid.
(137) The dispersion formulation according to (136), wherein the polyglyceryl fatty acid comprises polyglyceryl polyricinoleate.
(138) The dispersion formulation according to any one of (131) to (137), wherein the emulsifier comprises a sucrose fatty acid ester and a polyglyceryl fatty acid.
(139) The dispersion formulation according to any one of (131) to (138), wherein the water-soluble molecule comprises nicotinamide mononucleotide (NMN).

According to the present disclosure, the following inventions are provided.
(1001) A dispersion formulation comprising solid particles containing a complex of a water-soluble molecule and two or more types of emulsifiers, and an oily base, wherein said two or more types of emulsifiers comprise a polyglyceryl polyfatty acid having a number average degree of polymerization of 2 to 6 and an HLB value of 5 or less, and a sucrose fatty acid ester having an HLB value of 5 or less; the Z-average particle size of said solid particles is 100 nm or more and 500 nm or less; said solid particles are dispersed in said oily base; and said polyglyceryl polyfatty acid having a number average degree of polymerization of 2 to 6 and said sucrose fatty acid ester are contained in a ratio such that dispersibility is maintained transparently or translucently over 2 months under a temperature condition of 25°C.
(1002) The dispersion formulation according to (1001), wherein the glyceryl fatty acid is polyglyceryl polyricinoleate.
(1003) The dispersion formulation according to (1001), wherein the sucrose fatty acid ester comprises sucrose erucate ester.
(1004) The dispersion formulation according to (1002), wherein the sucrose fatty acid ester comprises sucrose erucate ester.
(1005) A cosmetic comprising the dispersion formulation according to any one of (1001) to (1004).
(1101) A dispersion formulation comprising solid particles containing a complex of a water-soluble molecule and two or more types of emulsifiers, and an oily base.
(1102) The dispersion formulation according to (1101), wherein said two or more types of emulsifiers each have an HLB value of 5 or less, preferably 4 or less.
(1103) The dispersion formulation according to (1101), wherein said two or more types of emulsifiers comprise at least a polyglyceryl polyfatty acid.
(1104) The dispersion formulation according to (1101), wherein said two or more types of emulsifiers comprise at least a sucrose fatty acid ester.
(1105) The dispersion formulation according to (1101), wherein said two or more types of emulsifiers comprise a polyglyceryl polyfatty acid and a sucrose fatty acid ester.
(1106) The dispersion formulation according to (1101), wherein said two or more types of emulsifiers comprise at least a polyglyceryl polyfatty acid having a number average degree of polymerization of 2 to 6 and having an HLB value of 5 or less.
(1107) The dispersion formulation according to (1101), wherein said two or more types of emulsifiers comprise at least a sucrose fatty acid ester having an HLB value of 5 or less.
(1108) The dispersion formulation according to (1101), wherein said two or more types of emulsifiers comprise a polyglyceryl polyfatty acid having a number average degree of polymerization of 2 to 6 and having an HLB value of 5 or less, and a sucrose fatty acid ester having an HLB value of 5 or less.
(1109) The dispersion formulation according to (1101), wherein said two or more types of emulsifiers comprise at least sucrose erucate ester.
(1110) The dispersion formulation according to (1101), wherein said two or more types of emulsifiers comprise at least sucrose erucate ester having an HLB value of 5 or less.
(1111) The dispersion formulation according to (1101), wherein said two or more types of emulsifiers comprise at least polyglyceryl polyricinoleate.
(1112) The dispersion formulation according to (1109), wherein said two or more types of emulsifiers comprise at least polyglyceryl polyricinoleate.
(1113) The dispersion formulation according to (1110), wherein said two or more types of emulsifiers comprise at least polyglyceryl polyricinoleate.
(1114) The dispersion formulation according to (1101), wherein said two or more types of emulsifiers comprise at least polyglyceryl polyricinoleate having an HLB value of 5 or less.
(1115) The dispersion formulation according to (1109), wherein said two or more types of emulsifiers comprise at least polyglyceryl polyricinoleate having an HLB value of 5 or less.
(1116) The dispersion formulation according to (1110), wherein said two or more types of emulsifiers comprise at least polyglyceryl polyricinoleate having an HLB value of 5 or less.
(1117) The dispersion formulation according to any one of the above, wherein said two or more types of emulsifiers each have a fatty chain having 18 to 22 carbon atoms.
(1118) The dispersion formulation according to any one of the above, wherein said two or more types of emulsifiers comprise a glyceryl fatty acid, and the glyceryl fatty acid has a fatty chain having 18 to 22 carbon atoms.
(1119) The dispersion formulation according to any one of the above, wherein said two or more types of emulsifiers comprise a fatty acid ester, and the fatty acid ester has a fatty chain having 18 to 22 carbon atoms.

### [Brief Description of the Drawings]

[FIG. 1A] Shows the degree of penetration into the stratum corneum of solid particle dispersion formulations prepared using an emulsifier described as ER290. The fractions in the figure indicate the ER290/emulsifier ratio.
[FIG. 1B] Same as above.
[FIG. 2A] Shows the degree of penetration into layers below the stratum corneum of solid particle dispersion formulations prepared using an emulsifier described as ER290. The fractions in the figure indicate the ER290/emulsifier ratio.
[FIG. 2B] Same as above.
[FIG. 3] Shows the appearance of the solid particle dispersion formulations of Examples 2-1 and 2-2.
[FIG. 4] Shows the particle size distribution of particles in the solid particle dispersion formulations of Examples 2-1 and 2-2.
[FIG. 5] Shows the penetration into the skin of the solid particle dispersion formulations of Examples 2-1 and 2-2.
[FIG. 6] Shows the particle size distribution of particles in the solid particle dispersion aqueous formulation of Example 3.
[FIG. 7] Shows the degree of penetration into the stratum corneum and layers below the stratum corneum of the solid particle dispersion aqueous formulation of Example 3.

### [Description of the Embodiments]

Embodiments according to the present invention will be described with reference to the drawings. Note that the present invention is not limited by the following embodiments and drawings. In the following embodiments, expressions such as "having," "including," or "containing" also encompass the meaning of "consisting of" or "composed of". The singular form means singular or plural.

As used herein, a "subject" is a mammal including a human, and is preferably a human. Examples of mammals include, but are not limited to, humans, chimpanzees and other primates; domestic animals, pet animals and laboratory animals such as dogs, cats, rabbits, horses, sheep, goats, cows, pigs, rats (including nude rats), mice (including nude mice and SCID mice), and guinea pigs.

As used herein, a "surfactant" is a molecule having a relatively hydrophilic moiety and a relatively hydrophobic moiety in the molecule. Surfactants are broadly classified into ionic surfactants and nonionic surfactants. Ionic surfactants include cationic surfactants and anionic surfactants. Typically, an ionic surfactant may have either a cation or an anion and a hydrophobic moiety. The cationic surfactant may contain a cationic moiety and a fatty chain (e.g., a long fatty chain) such as alkyl, alkenyl, or alkynyl. The anionic surfactant may contain an anionic moiety and a fatty acid such as a long-chain fatty acid, an unsaturated fatty acid (e.g., monovalent or divalent), or a long-chain unsaturated fatty acid (e.g., monovalent or divalent). "Long-chain" is a term given to compounds having 14 to 22 carbon atoms. The fatty chain may be a fatty chain having 8 to 22 carbon atoms, i.e., a C8-C22 alkyl, C8-C22 alkenyl, or C8-C22 alkynyl. Also, the fatty chain can be a long-chain fatty chain. Saturated means that the fatty chain has no double bonds or triple bonds, and unsaturated means that the fatty chain has at least one double bond or triple bond. Fatty chains having a double bond include cis-type and trans-type, but the cis-type is considered preferable from the viewpoint of biocompatibility. Nonionic surfactants are surfactants that do not have an ionic group in the molecule, and have a nonionic hydrophilic moiety and a hydrophobic moiety. The hydrophobic moiety may contain a fatty chain (e.g., a long fatty chain) such as alkyl, alkenyl, or alkynyl. Fatty chains and long-chain fatty chains are as defined above.

As the nonionic surfactant, ester compounds made from fatty acids (e.g., saturated fatty acids or unsaturated fatty acids) such as erucic acid and oleic acid are preferable. Examples of lipophilic nonionic surfactants include sucrose fatty acid esters having a high degree of esterification (i.e., a high ratio of di-, tri-, and polyesters relative to monoesters), preferably sucrose fatty acid esters (sucrose stearate, sucrose palmitate, sucrose myristate, sucrose oleate, sucrose laurate, sucrose erucate ester, sucrose mixed fatty acid esters), polyglycerin condensed ricinoleate, decaglycerin esters, glycerin fatty acid esters, polyglycerin fatty acid esters, polyoxyethylene glycerin fatty acid esters, sorbitan fatty acid esters, polyoxyethylene sorbitol fatty acid esters, and polyoxyethylene castor oil/hydrogenated castor oil. Suitably, the nonionic surfactant is sucrose laurate. The surfactants may be used alone or in combination of two or more. Suitable examples of surfactants used for the preparation of the complex are lipophilic (hydrophobic) nonionic surfactants having an HLB (Hydrophile-Lipophile Balance) value of 10 or less. The HLB of each nonionic surfactant can be, for example, 8 or less, 7 or less, 6 or less, 5 or less, 4.9 or less, 4.8 or less, 4.7 or less, 4.6 or less, 4.5 or less, 4.4 or less, 4.3 or less, 4.2 or less, 4.1 or less, 4.0 or less, 3.9 or less, 3.8 or less, 3.7 or less, 3.6 or less, 3.5 or less, 3.4 or less, 3.3 or less, 3.2 or less, 3.1 or less, or 3 or less. The HLB of the nonionic surfactant is preferably 8 or less, more preferably 5 or less, even more preferably 4 or less, and particularly preferably 3 or less.

Cationic surfactants (cationic lipids) are described in U.S. Patent Application Publication Nos. 2006/0083780 and 2006/0240554, U.S. Patent Nos. 5,208,036, 5,264,618, 5,279,833, 5,283,185, 5,753,613, and 5,785,992, and PCT Publication No. WO96/10390, the disclosures of which are incorporated herein by reference in their entirety for all purposes. Examples of cationic lipids include 1,2-dilinoleyloxy-N,N-dimethylaminopropane (DLinDMA), 1,2-dilinolenyloxy-N,N-dimethylaminopropane (DLenDMA), 2,2-dilinoleyl-4-(2-dimethylaminoethyl)--dioxolane (DLin-K-C2-DMA; "XTC2"), 2,2-dilinoleyl-4-(3-dimethylaminopropyl)--dioxolane (DLin-K-C3-DMA), 2,2-dilinoleyl-4-(4-dimethylaminobutyl)--dioxolane (DLin-K-C4-DMA), 2,2-dilinoleyl-5-dimethylaminomethyl--dioxane (DLin-K6-DMA), 2,2-dilinoleyl-4-N-methylpepiazino--dioxolane (DLin-K-MPZ), 2,2-dilinoleyl-4-dimethylaminomethyl-dioxolane (DLin-K-DMA), 1,2-dilinoleylcarbamoyloxy-3-dimethylaminopropane (DLin-C-DAP), 1,2-dilinoleyloxy-3-(dimethylamino)acetoxypropane (DLin-DAC), 1,2-dilinoleyloxy-3-morpholinopropane (DLin-MA), 1,2-dilinoleoyl-3-dimethylaminopropane (DLinDAP), 1,2-dilinoleylthio-3-dimethylaminopropane (DLin-S-DMA), 1-linoleoyl-2-linoleyloxy-3-dimethylaminopropane (DLin-2-DMAP), 1,2-dilinoleyloxy-3-trimethylaminopropane chloride salt (DLin-TMA.Cl), 1,2-dilinoleoyl-3-trimethylaminopropane chloride salt (DLin-TAP.Cl), 1,2-dilinoleyloxy-3-(N-methylpiperazino)propane (DLin-MPZ), 3-(N,N-dilinoleylamino)-1,2-propanediol (DLinAP), 3-(N,N-dioleylamino)-1,2-propanediol (DOAP), 1,2-dilinoleyloxo-3-(2-N,N-dimethylamino)ethoxypropane (DLin-EG-DMA), N,N-dioleyl-N,N-dimethylammonium chloride (DODAC), 1,2-dioleyloxy-N,N-dimethylaminopropane (DODMA), 1,2-distearyloxy-N,N-dimethylaminopropane (DSDMA), N-(1-(2,3-dioleyloxy)propyl)-N,N,N-trimethylammonium chloride (DOTMA), N,N-distearyl-N,N-dimethylammonium bromide (DDAB), N-(1-(2,3-dioleoyloxy)propyl)-N,N,N-trimethylammonium chloride (DOTAP), 3-(N-(N',N'-dimethylaminoethane)-carbamoyl)cholesterol (DC-Chol), N-(1,2-dimyristyloxyprop-3-yl)-N,N-dimethyl-N-hydroxyethylammonium bromide (DMRIE), 2,3-dioleyloxy-N-[2(spermine-carboxamido)ethyl]-N,N-dimethyl-1-propanaminium trifluoroacetate (DOSPA), dioctadecylamidoglycylspermine (DOGS), 3-dimethylamino-2-(cholest-5-en-3-beta-oxybutan-4-oxy)-1-(cis,cis-9,12-octadecadienoxy)propane (CLinDMA), 2-[5'-(cholest-5-en-3-beta-oxy)-3'-oxapentoxy)-3-dimethyl-1-(cis,cis-9',1-2'-octadecadienoxy) propane (CpLinDMA), N,N-dimethyl-3,4-dioleyloxybenzylamine (DMOBA), 1,2-N,N'-dioleylcarbamyl-3-dimethylaminopropane (DOcarbDAP), 1,2-N,N'-dilinoleylcarbamyl-3-dimethylaminopropane (DLincarbDAP), or mixtures thereof. In certain preferred embodiments, the cationic lipid is DLinDMA, DLin-K-C2-DMA ("XTC2"), or a mixture thereof. The cationic lipid preferably includes 1,2-dimyristoyl-sn-glycero-3-ethyl-phosphatidylcholine (EDMPC).

Anionic surfactants (anionic lipids) include phospholipids and fatty acids. Examples of anionic surfactants include phosphatidylglycerol, cardiolipin, diacylphosphatidylserine, diacylphosphatidic acid, N-dodecanoyl phosphatidylethanolamine, N-succinyl phosphatidylethanolamine, N-glutaryl phosphatidylethanolamine, lysylphosphatidylglycerol, palmitoyloleoylphosphatidylglycerol (POPG), and those in which other anionic modifying groups are bonded to neutral lipids.

As used herein, a "solid complex" is a complex consisting of solid content. In the present specification, a solid complex in the form of particles may be referred to as solid particles. The solid complex may contain water, but is a complex containing solid components. The solid complex may have a low water content, for example, a water content of 1% by mass or less, 0.9% by mass or less, 0.8% by mass or less, 0.7% by mass or less, 0.6% by mass or less, 0.5% by mass or less, 0.4% by mass or less, 0.3% by mass or less, 0.2% by mass or less, or 0.1% by mass or less, as measured by a known method. A solid complex comprising a water-soluble molecule and a surfactant can have a core-shell structure, wherein the core comprises the water-soluble molecule and the shell comprises the surfactant, and the surfactant can direct the water-soluble moiety toward the core and the hydrophobic moiety toward the outer surface of the solid complex. Thus, the resulting complex or solid complex may have a hydrophilic center (core) but will have a hydrophobic outer surface (periphery or shell). Such solid complexes can be preferably dispersed in an oil phase. However, in the present disclosure, such solid complexes need not be dispersed in an oil phase (or liquid). By not using an oil phase, it is possible to prevent the skin from becoming excessively oily due to oil. Also, by not using an oil phase, handling of the patch may be facilitated in some cases.

### (Dispersion Formulation)

According to the present disclosure, a dispersion formulation comprising solid particles containing a complex of a water-soluble molecule and an emulsifier, and an oily base is provided. In the dispersion formulation, the solid particles are dispersed in the oily base.

According to the present disclosure, a dispersion formulation comprising solid particles containing a complex of a water-soluble molecule and an emulsifier, and an aqueous base is provided. In the dispersion formulation, the solid particles are dispersed in the aqueous base.

In certain preferred embodiments, the emulsifier comprises one or more emulsifiers. In certain preferred embodiments, the emulsifier comprises two or more emulsifiers.

In certain preferred embodiments, the emulsifier includes an emulsifier having a fatty chain with 18 to 22 carbon atoms as the fatty chain. In certain preferred embodiments, each of the two or more emulsifiers includes an emulsifier having a fatty chain with 18 to 22 carbon atoms as the fatty chain. The two or more emulsifiers may further include any additional emulsifier as long as they include two emulsifiers having a fatty chain with 18 to 22 carbon atoms as the fatty chain.

In certain preferred embodiments, the emulsifier preferably comprises a sucrose fatty acid ester. In certain preferred embodiments, the sucrose fatty acid ester is a sucrose fatty acid ester, and more preferably sucrose erucate ester.

In certain preferred embodiments, the emulsifier comprises a glyceryl fatty acid. The glyceryl fatty acid is selected from the group consisting of, for example, polyglyceryl polyfatty acid, polyglyceryl monofatty acid, monoglyceryl polyfatty acid, and monoglyceryl monofatty acid.

In certain preferred embodiments, the glyceryl fatty acid has a polyglyceryl group with a degree of polymerization of 2 to 10. In certain preferred embodiments, the glyceryl fatty acid has a polyglyceryl group with a degree of polymerization of 2 to 8, or 2 to 6, 2 to 4, 3 to 5, or 4 to 6.

In certain preferred embodiments, examples of the glyceryl fatty acid include, but are not particularly limited to, Glyceryl Fatty Acid Group A: polyglyceryl decaoleate, polyglyceryl polyricinoleate, polyglyceryl polyricinoleate, polyglyceryl diisostearate, polyglyceryl tetraisostearate, glyceryl monooleate, polyoxyethylene hydrogenated castor oil, polyglyceryl pentaoleate, polyglyceryl decaisostearate, and glyceryl monocaprate. In certain preferred embodiments, examples of the glyceryl fatty acid include, but are not particularly limited to, Glyceryl Fatty Acid Group B: polyglyceryl-10 decaoleate, polyglyceryl-6 polyricinoleate, polyglyceryl-6 polyricinoleate, polyglyceryl-2 diisostearate, polyglyceryl-2 tetraisostearate, glyceryl monooleate, polyoxyethylene hydrogenated castor oil, polyglyceryl-10 pentaoleate, polyglyceryl-10 decaisostearate, and glyceryl monocaprate.

In certain preferred embodiments, the emulsifier comprises a sucrose fatty acid ester and a glyceryl fatty acid.

In certain preferred specific embodiments, the emulsifier comprises sucrose erucate ester and a glyceryl fatty acid. In certain preferred embodiments, the glyceryl fatty acid is selected from the group consisting of polyglyceryl polyfatty acid, polyglyceryl monofatty acid, monoglyceryl polyfatty acid, and monoglyceryl monofatty acid. In certain preferred embodiments, the glyceryl fatty acid may be, but is not particularly limited to, for example, one or more selected from Glyceryl Fatty Acid Group A. In certain preferred embodiments, the glyceryl fatty acid may be, but is not particularly limited to, for example, one or more selected from Glyceryl Fatty Acid Group B.

In certain preferred specific embodiments, the emulsifier comprises sucrose erucate ester and polyglyceryl-10 decaoleate. The molar ratio of sucrose erucate ester to polyglyceryl-10 decaoleate can be, for example, 5:95 to 95:5, 10:90 to 90:10, 15:85 to 85:15, 20:80 to 80:20, 25:75 to 75:25, 30:70 to 70:30, 35:65 to 65:35, or 40:60 to 60:40. The ratio of sucrose erucate ester to polyglyceryl-10 decaoleate can be, for example, 5:95 to 75:25, 10:90 to 65:35, 15:85 to 50:50, 20:80 to 40:60, 20:80 to 35:65, or 20:80 to 30:70. The ratio of sucrose erucate ester to polyglyceryl-10 decaoleate can be, for example, 25:75 to 95:5, 35:65 to 90:10, 50:50 to 85:15, 60:40 to 80:20, 65:35 to 80:20, or 70:30 to 80:20.

In certain preferred specific embodiments, the emulsifier comprises sucrose erucate ester and polyglyceryl-6 polyricinoleate. The ratio of sucrose erucate ester to polyglyceryl-6 polyricinoleate can be, for example, 5:95 to 95:5, 10:90 to 90:10, 15:85 to 85:15, 20:80 to 80:20, 25:75 to 75:25, 30:70 to 70:30, 35:65 to 65:35, or 40:60 to 60:40. The ratio of sucrose erucate ester to polyglyceryl-6 polyricinoleate can be, for example, 5:95 to 75:25, 10:90 to 65:35, 15:85 to 50:50, 20:80 to 40:60, 20:80 to 35:65, or 20:80 to 30:70. The ratio of sucrose erucate ester to polyglyceryl-6 polyricinoleate can be, for example, 25:75 to 95:5, 35:65 to 90:10, 50:50 to 85:15, 60:40 to 80:20, 65:35 to 80:20, or 70:30 to 80:20.

In certain preferred embodiments, the emulsifier comprises sucrose erucate ester and polyglyceryl-6 polyricinoleate. The ratio of sucrose erucate ester to polyglyceryl-6 polyricinoleate can be, for example, 5:95 to 95:5, 10:90 to 90:10, 15:85 to 85:15, 20:80 to 80:20, 25:75 to 75:25, 30:70 to 70:30, 35:65 to 65:35, or 40:60 to 60:40. The ratio of sucrose erucate ester to polyglyceryl-6 polyricinoleate can be, for example, 5:95 to 75:25, 10:90 to 65:35, 15:85 to 50:50, 20:80 to 40:60, 20:80 to 35:65, or 20:80 to 30:70. The ratio of sucrose erucate ester to polyglyceryl-6 polyricinoleate can be, for example, 25:75 to 95:5, 35:65 to 90:10, 50:50 to 85:15, 60:40 to 80:20, 65:35 to 80:20, or 70:30 to 80:20.

In certain preferred embodiments, the emulsifier comprises sucrose erucate ester and polyglyceryl-2 diisostearate. The ratio of sucrose erucate ester to polyglyceryl-2 diisostearate can be, for example, 5:95 to 95:5, 10:90 to 90:10, 15:85 to 85:15, 20:80 to 80:20, 25:75 to 75:25, 30:70 to 70:30, 35:65 to 65:35, or 40:60 to 60:40. The ratio of sucrose erucate ester to polyglyceryl-2 diisostearate can be, for example, 5:95 to 75:25, 10:90 to 65:35, 15:85 to 50:50, 20:80 to 40:60, 20:80 to 35:65, or 20:80 to 30:70. The ratio of sucrose erucate ester to polyglyceryl-2 diisostearate can be, for example, 25:75 to 95:5, 35:65 to 90:10, 50:50 to 85:15, 60:40 to 80:20, 65:35 to 80:20, or 70:30 to 80:20.

In certain preferred embodiments, the emulsifier comprises sucrose erucate ester and polyglyceryl-2 tetraisostearate. The ratio of sucrose erucate ester to polyglyceryl-2 tetraisostearate can be, for example, 5:95 to 95:5, 10:90 to 90:10, 15:85 to 85:15, 20:80 to 80:20, 25:75 to 75:25, 30:70 to 70:30, 35:65 to 65:35, or 40:60 to 60:40. The ratio of sucrose erucate ester to polyglyceryl-2 tetraisostearate can be, for example, 5:95 to 75:25, 10:90 to 65:35, 15:85 to 50:50, 20:80 to 40:60, 20:80 to 35:65, or 20:80 to 30:70. The ratio of sucrose erucate ester to polyglyceryl-2 tetraisostearate can be, for example, 25:75 to 95:5, 35:65 to 90:10, 50:50 to 85:15, 60:40 to 80:20, 65:35 to 80:20, or 70:30 to 80:20.

In certain preferred embodiments, the emulsifier comprises sucrose erucate ester and glyceryl monooleate. The ratio of sucrose erucate ester to glyceryl monooleate can be, for example, 5:95 to 95:5, 10:90 to 90:10, 15:85 to 85:15, 20:80 to 80:20, 25:75 to 75:25, 30:70 to 70:30, 35:65 to 65:35, or 40:60 to 60:40. The ratio of sucrose erucate ester to glyceryl monooleate can be, for example, 5:95 to 75:25, 10:90 to 65:35, 15:85 to 50:50, 20:80 to 40:60, 20:80 to 35:65, or 20:80 to 30:70. The ratio of sucrose erucate ester to glyceryl monooleate can be, for example, 25:75 to 95:5, 35:65 to 90:10, 50:50 to 85:15, 60:40 to 80:20, 65:35 to 80:20, or 70:30 to 80:20.

In certain preferred embodiments, the emulsifier comprises sucrose erucate ester and polyoxyethylene hydrogenated castor oil. The ratio of sucrose erucate ester to polyoxyethylene hydrogenated castor oil can be, for example, 5:95 to 95:5, 10:90 to 90:10, 15:85 to 85:15, 20:80 to 80:20, 25:75 to 75:25, 30:70 to 70:30, 35:65 to 65:35, or 40:60 to 60:40. The ratio of sucrose erucate ester to polyoxyethylene hydrogenated castor oil can be, for example, 5:95 to 75:25, 10:90 to 65:35, 15:85 to 50:50, 20:80 to 40:60, 20:80 to 35:65, or 20:80 to 30:70. The ratio of sucrose erucate ester to polyoxyethylene hydrogenated castor oil can be, for example, 25:75 to 95:5, 35:65 to 90:10, 50:50 to 85:15, 60:40 to 80:20, 65:35 to 80:20, or 70:30 to 80:20.

In certain preferred embodiments, the emulsifier comprises sucrose erucate ester and polyglyceryl-10 pentaoleate. The ratio of sucrose erucate ester to polyglyceryl-10 pentaoleate can be, for example, 5:95 to 95:5, 10:90 to 90:10, 15:85 to 85:15, 20:80 to 80:20, 25:75 to 75:25, 30:70 to 70:30, 35:65 to 65:35, or 40:60 to 60:40. The ratio of sucrose erucate ester to polyglyceryl-10 pentaoleate can be, for example, 5:95 to 75:25, 10:90 to 65:35, 15:85 to 50:50, 20:80 to 40:60, 20:80 to 35:65, or 20:80 to 30:70. The ratio of sucrose erucate ester to polyglyceryl-10 pentaoleate can be, for example, 25:75 to 95:5, 35:65 to 90:10, 50:50 to 85:15, 60:40 to 80:20, 65:35 to 80:20, or 70:30 to 80:20.

In certain preferred embodiments, the emulsifier comprises sucrose erucate ester and polyglyceryl-10 decaisostearate. The ratio of sucrose erucate ester to polyglyceryl-10 decaisostearate can be, for example, 5:95 to 95:5, 10:90 to 90:10, 15:85 to 85:15, 20:80 to 80:20, 25:75 to 75:25, 30:70 to 70:30, 35:65 to 65:35, or 40:60 to 60:40. The ratio of sucrose erucate ester to polyglyceryl-10 decaisostearate can be, for example, 5:95 to 75:25, 10:90 to 65:35, 15:85 to 50:50, 20:80 to 40:60, 20:80 to 35:65, or 20:80 to 30:70. The ratio of sucrose erucate ester to polyglyceryl-10 decaisostearate can be, for example, 25:75 to 95:5, 35:65 to 90:10, 50:50 to 85:15, 60:40 to 80:20, 65:35 to 80:20, or 70:30 to 80:20.

In certain preferred embodiments, the emulsifier comprises sucrose erucate ester and glyceryl monocaprate. The ratio of sucrose erucate ester to glyceryl monocaprate can be, for example, 5:95 to 95:5, 10:90 to 90:10, 15:85 to 85:15, 20:80 to 80:20, 25:75 to 75:25, 30:70 to 70:30, 35:65 to 65:35, or 40:60 to 60:40. The ratio of sucrose erucate ester to glyceryl monocaprate can be, for example, 5:95 to 75:25, 10:90 to 65:35, 15:85 to 50:50, 20:80 to 40:60, 20:80 to 35:65, or 20:80 to 30:70. The ratio of sucrose erucate ester to glyceryl monocaprate can be, for example, 25:75 to 95:5, 35:65 to 90:10, 50:50 to 85:15, 60:40 to 80:20, 65:35 to 80:20, or 70:30 to 80:20.

In the present disclosure, the oily base is not particularly limited as long as it is liquid at room temperature (e.g., 25°C), substantially insoluble in water, viscous, has a specific gravity lower than that of water, is used in the fields of cosmetics and pharmaceuticals, and does not cause adverse effects when contacting the skin of animals including humans. Examples of such oily bases include higher (polyhydric) alcohols such as cetanol, myristyl alcohol, oleyl alcohol, lauryl alcohol, cetostearyl alcohol, stearyl alcohol, behenyl alcohol, jojoba alcohol, chimyl alcohol, selachyl alcohol, batyl alcohol, hexyldecanol, isostearyl alcohol, 2-octyldodecanol, and dimer diol; aralkyl alcohols such as benzyl alcohol and derivatives thereof; long-chain fatty acids and derivatives thereof such as isostearic acid, behenic acid, undecylenic acid, 12-hydroxystearic acid, palmitoleic acid, oleic acid, linoleic acid, linolenic acid, erucic acid, docosahexaenoic acid, eicosapentaenoic acid, isohexadecanoic acid, anteisoheneicosanoic acid, dimer acid, and hydrogenated dimer acid; hydrocarbons such as liquid paraffin (mineral oil), heavy liquid isoparaffin, light liquid isoparaffin, alpha-olefin oligomer, polyisobutene, hydrogenated polyisobutene, polybutene, squalane, olive-derived squalane, squalene, petrolatum, and solid paraffin; waxes such as candelilla wax, carnauba wax, rice wax, Japan wax, beeswax, montan wax, ozokerite, ceresin, paraffin wax, microcrystalline wax, petrolatum, Fischer-Tropsch wax, polyethylene wax, and ethylene-propylene copolymer; vegetable oils and fats such as coconut oil, palm oil, palm kernel oil, safflower oil, olive oil, castor oil, avocado oil, sesame oil, tea oil, evening primrose oil, wheat germ oil, perilla oil, apricot kernel oil, almond oil, macadamia nut oil, hazelnut oil, kukui nut oil, rosehip oil, meadowfoam oil, persic oil, tea tree oil, peppermint oil, corn oil, rapeseed oil, sunflower oil, wheat germ oil, linseed oil, cottonseed oil, soybean oil, peanut oil, rice bran oil, cacao butter, shea butter, hydrogenated coconut oil, hydrogenated castor oil, jojoba oil, and hydrogenated jojoba oil; animal oils and fats such as beef tallow, milk fat, horse fat, egg yolk oil, mink oil, and turtle oil; animal waxes such as spermaceti, lanolin, and orange roughy oil; lanolins such as liquid lanolin, reduced lanolin, adsorption-purified lanolin, lanolin acetate, liquid lanolin acetate, hydroxylanolin, polyoxyethylene lanolin, lanolin fatty acid, hard lanolin fatty acid, lanolin alcohol, lanolin alcohol acetate, and acetic acid (cetyl/lanolyl) ester; sterols such as cholesterol, dihydrocholesterol, lanosterol, dihydrolanosterol, phytosterol, and cholic acid; sapogenins; saponins; sterol esters such as cholesteryl acetate, cholesteryl nonanoate, cholesteryl stearate, cholesteryl isostearate, cholesteryl oleate, di(cholesteryl/behenyl/octyldodecyl) N-lauroyl-L-glutamate, di(cholesteryl/octyldodecyl) N-lauroyl-L-glutamate, di(phytosteryl/behenyl/octyldodecyl) N-lauroyl-L-glutamate, di(phytosteryl/octyldodecyl) N-lauroyl-L-glutamate, cholesteryl 12-hydroxystearate, cholesteryl macadamia nut oil fatty acid, phytosteryl macadamia nut oil fatty acid, phytosteryl isostearate, soft lanolin fatty acid cholesteryl, hard lanolin fatty acid cholesteryl, long-chain branched fatty acid cholesteryl, and long-chain alpha-hydroxy fatty acid cholesteryl; acyl sarcosine alkyl esters such as isopropyl N-lauroyl sarcosinate; lipid complexes such as phospholipid-cholesterol complexes and phospholipid-phytosterol complexes; long-chain fatty acid esters such as octyldodecyl myristate, hexyldecyl myristate, octyldodecyl isostearate, cetyl palmitate, octyldodecyl palmitate, cetyl octanoate, hexyldecyl octanoate, isotridecyl isononanoate, isononyl isononanoate, octyl isononanoate, isotridecyl isononanoate, isodecyl neopentanoate, isotridecyl neopentanoate, isostearyl neopentanoate, octyldodecyl neodecanoate, oleyl oleate, octyldodecyl oleate, octyldodecyl ricinoleate, octyldodecyl lanolin fatty acid, hexyldecyl dimethyloctanoate, octyldodecyl erucate, hydrogenated castor oil isostearate, ethyl oleate, ethyl avocado oil fatty acid, isopropyl myristate, isopropyl palmitate, octyl palmitate, isopropyl isostearate, and isopropyl lanolin fatty acid; dicarboxylic acid esters such as diethyl sebacate, diisopropyl sebacate, dioctyl sebacate, diisopropyl adipate, dibutyloctyl sebacate, diisobutyl adipate, dioctyl succinate, and triethyl citrate; oxyacid esters such as cetyl lactate, diisostearyl malate, and hydrogenated castor oil monoisostearate; polyhydric alcohol fatty acid esters such as glyceryl trioctanoate (glyceryl tri-2-ethylhexanoate), glyceryl trioleate, glyceryl triisostearate, glyceryl diisostearate, glyceryl tri(caprylate/caprate), glyceryl tri(caprylate/caprate/myristate/stearate), hydrogenated rosin triglyceride (hydrogenated ester gum), rosin triglyceride (ester gum), glyceryl behenate eicosadioate, trimethylolpropane trioctanoate, trimethylolpropane triisostearate, neopentyl glycol dioctanoate, neopentyl glycol dicaprate, 2-butyl-2-ethyl-1,3-propanediol dioctanoate, propylene glycol dioleate, pentaerythrityl tetraoctanoate, pentaerythrityl hydrogenated rosin, ditrimethylolpropane triethylhexanoate, ditrimethylolpropane (isostearate/sebacate), pentaerythrityl triethylhexanoate, dipentaerythrityl (hydroxystearate/stearate/rosinate), diglyceryl diisostearate, polyglyceryl tetraisostearate, polyglyceryl-10 nonaisostearate, polyglyceryl-8 deca(erucate/isostearate/ricinoleate), diglyceryl oligoester (hexyldecanoate/sebacate), glycol distearate (ethylene glycol distearate), 3-methyl-1,5-pentanediol dineopentanoate, and 2,4-diethyl-1,5-pentanediol dineopentanoate; derivatives of dimer acids or dimer diols such as diisopropyl dimer dilinoleate, diisostearyl dimer dilinoleate, di(isostearyl/phytosteryl) dimer dilinoleate, (phytosteryl/behenyl) dimer dilinoleate, (phytosteryl/isostearyl/cetyl/stearyl/behenyl) dimer dilinoleate, dimer dilinoleyl dimer dilinoleate, dimer dilinoleyl diisostearate, dimer dilinoleyl hydrogenated rosin condensate, dimer dilinoleic acid hydrogenated castor oil, and hydroxyalkyl dimer dilinoleyl ether; fatty acid alkanolamides such as coconut oil fatty acid monoethanolamide (Cocamide MEA), coconut oil fatty acid diethanolamide (Cocamide DEA), lauric acid monoethanolamide (Lauramide MEA), lauric acid diethanolamide (Lauramide DEA), lauric acid monoisopropanolamide (Lauramide MIPA), palmitic acid monoethanolamide (Paltamide MEA), palmitic acid diethanolamide (Paltamide DEA), and coconut oil fatty acid methylethanolamide (Cocamide Methyl MEA); silicones such as cyclomethicone (cyclic dimethylsiloxane) including dimethicone (dimethylpolysiloxane), highly polymerized dimethicone (highly polymerized dimethylpolysiloxane), decamethylcyclopentasiloxane (also simply referred to as cyclopentasiloxane), phenyl trimethicone, diphenyl dimethicone, phenyl dimethicone, stearoxypropyldimethylamine, (aminoethylaminopropylmethicone/dimethicone) copolymer, dimethiconol, dimethiconol crosspolymer, silicone resin, silicone rubber, amino-modified silicones such as aminopropyl dimethicone and amodimethicone, cation-modified silicones, polyether-modified silicones such as dimethicone copolyol, polyglycerin-modified silicones, sucrose-modified silicones, carboxylic acid-modified silicones, phosphoric acid-modified silicones, sulfuric acid-modified silicones, alkyl-modified silicones, fatty acid-modified silicones, alkyl ether-modified silicones, amino acid-modified silicones, peptide-modified silicones, fluorine-modified silicones, cation-modified and polyether-modified silicones, amino-modified and polyether-modified silicones, alkyl-modified and polyether-modified silicones, and polysiloxane-oxyalkylene copolymers; and fluorine-based oils such as perfluorodecane, perfluorooctane, and perfluoropolyether. Among these, long-chain fatty acids such as oleic acid, vegetable oils and fats such as olive oil and jojoba oil, alkyl esters having 1 to 8 carbon atoms of fatty acids having 12 to 18 carbon atoms such as isopropyl myristate, isopropyl palmitate, octyl palmitate, and isopropyl isostearate, and hydrocarbons such as squalane are preferable, and isopropyl myristate and squalane are more preferable. Particularly preferably, the oily base can be isopropyl myristate.

As the aqueous base, water or an aqueous solution containing a pharmaceutically acceptable additive can be used. Examples of pharmaceutically acceptable additives include pharmaceutically acceptable salts, isotonic agents, pH adjusters, thickeners, and the like. The aqueous solution containing a pharmaceutically acceptable additive can be, for example, physiological saline.

Examples of the water-soluble molecule include polysaccharides such as hyaluronic acid and its salts, heparin and its analogous compounds, and chitosan; proteins such as ovalbumin, casein, and collagen; vitamins such as vitamin C and its derivatives, and retinol; amino acids such as amino acids, tranexamic acid, and placenta extract; glycosides such as arbutin; coenzymes such as coenzyme Q10; nucleotides such as nicotinamide mononucleotide (NMN); protein preparations such as cholera toxin subunit B, epidermal growth factor, serum albumin, immunoglobulin, interleukin, interferon, human glucagon, human growth hormone, erythropoietin, platelet-derived growth factor, and insulin; and peptide preparations such as cancer vaccines and allergy vaccines. The molecular weight of the water-soluble molecule is preferably 500 Da or more, more preferably 1000 Da (i.e., 1 kDa) or more, and preferably 200 kDa or less, more preferably 100 kDa or less, and particularly preferably 50 kDa or less.

The water-soluble molecule can be nanoparticles or nanovesicles, such as lipid vesicles, lipid nanoparticles, extracellular vesicles (e.g., exosomes), polyion complex micelles, and polyion complex polymersomes. Nanoparticles or nanovesicles refer to those having a Z-average size of sub-micrometers. Even when such nanoparticles or nanovesicles are used as the water-soluble molecule, the manufacturing process of the solid particle dispersion formulation can be applied. Nanoparticles or nanovesicles as described above can be mixed with an emulsifier in a volatile oil phase (e.g., cyclohexane) to form an emulsion, and the volatile oil phase can be removed by freeze-drying.

The particle size (Z-average size) of the solid particles can be, for example, 700 nm or less, 600 nm or less, 500 nm or less, 400 nm or less, 300 nm or less, or 200 nm or less. The particle size (Z-average size) of the solid particles can be, for example, 100 nm or more or 200 nm or more. The particle size (Z-average size) of the solid particles can be, for example, 100 nm to 600 nm, 100 nm to 500 nm, 100 nm to 400 nm, 100 nm to 300 nm, 100 nm to 200 nm, 200 nm to 600 nm, 200 nm to 500 nm, 200 nm to 400 nm, or 200 nm to 300 nm. The Z-average size is defined in ISO 13321. Particles smaller than 2 µm in diameter can be measured by dynamic light scattering (DLS) using a Zetasizer Nano ZS 90 (Malvern Instr. Ltd.).

The polydispersity index (PDI) of the solid particles can be, for example, 0.1 to 1. The PDI can be preferably 0.1 to 0.5, 0.1 to 0.4, 0.1 to 0.3, or 0.1 to 0.2.

In certain preferred embodiments, the dispersion formulation of the present disclosure may have a Z-average particle size at 20°C of 500 nm or less and 100 nm or more using a Zetasizer (e.g., Zetasizer Nano Pro), even after being left to stand at 25°C in the atmosphere for 2 months after preparation. Before measuring the Z particle size, the dispersion formulation of the present disclosure may be diluted (e.g., 100-fold dilution) with an oily base such as cyclohexane.

In certain preferred embodiments, the dispersion formulation of the present disclosure has no precipitation of solid particles at the time of preparation. In a more preferred embodiment, the dispersion formulation of the present disclosure has no precipitation of solid particles at the time of preparation, and further, the solid particles are dispersed transparently or translucently in the dispersion medium. This dispersibility can be adjusted by the mixing ratio of two or more kinds of emulsifiers. Therefore, the dispersion formulation of the present disclosure preferably contains two or more kinds of emulsifiers in a ratio such that dispersibility is maintained transparently or translucently.

The dispersion formulation of the present disclosure contains two or more kinds of emulsifiers, and preferably can have improved storage stability. In certain preferred embodiments, the dispersion formulation of the present disclosure does not have precipitation of solid particles even after being left to stand at 25°C in the atmosphere for a certain period of time after preparation. In a more preferred embodiment, the dispersion formulation of the present disclosure does not have precipitation of solid particles even after being left to stand at 25°C in the atmosphere for a certain period of time after preparation, and further, the solid particles are dispersed transparently or translucently in the dispersion medium. This dispersibility can be adjusted by the mixing ratio of two or more kinds of emulsifiers. Therefore, the dispersion formulation of the present disclosure preferably contains two or more kinds of emulsifiers in a ratio such that dispersibility is maintained transparently or translucently over a certain period of time under a temperature condition of 25°C. The certain period of time can be 10 days or more, 20 days or more, 1 month or more, 40 days or more, 50 days or more, or 2 months or more. The certain period of time can also be, for example, 3 months or less or 2 months or less. The certain period of time can be, for example, about 2 months.

The dispersion formulation of the present disclosure contains two or more kinds of emulsifiers, and preferably can have improved storage stability. In certain preferred embodiments, the dispersion formulation of the present disclosure does not have precipitation of solid particles even after being left to stand at 25°C in the atmosphere for 2 months after preparation. In a more preferred embodiment, the dispersion formulation of the present disclosure does not have precipitation of solid particles even after being left to stand at 25°C in the atmosphere for 2 months after preparation, and further, the solid particles are dispersed transparently or translucently in the dispersion medium. This dispersibility can be adjusted by the mixing ratio of two or more kinds of emulsifiers. Therefore, the dispersion formulation of the present disclosure preferably contains two or more kinds of emulsifiers in a ratio such that dispersibility is maintained transparently or translucently over 2 months under a temperature condition of 25°C.

### (Method for Preparing Dispersion Formulation)

When a target molecule such as a water-soluble molecule is mixed with an emulsifier in an oil phase (preferably in a volatile oil phase, for example, cyclohexane), particles having a core-shell structure containing the target molecule such as a water-soluble molecule in the core and the emulsifier in the shell are formed. The particles are dispersed in the oil phase. The emulsifier comprises one or more emulsifiers. If the oil phase used here is a volatile oil phase (e.g., cyclohexane), it is easy to subsequently evaporate the solvent, and by removing the solvent by drying (e.g., freeze-drying), particles having a core-shell structure containing the water-soluble molecule in the core and the emulsifier in the shell can be obtained as solid particles (also referred to as "solid complex"). By dispersing the solid particles in an oily base or an aqueous base, a solid particle dispersion oily formulation or a solid particle dispersion aqueous formulation (also simply referred to as "solid particle dispersion formulation") can be obtained, respectively. Such a solid particle dispersion oily formulation can penetrate deeply into the stratum corneum (which is non-hydrophilic), where water-soluble molecules typically have difficulty penetrating, and in some cases can penetrate below the stratum corneum, preferably penetrating into the blood. According to the examples, the solid particle dispersion aqueous formulation also has the ability to permeate active ingredients below the stratum corneum, preferably penetrating into the blood.

### (Cosmetics)

According to the present disclosure, a cosmetic comprising the dispersion formulation of the present disclosure is provided. The cosmetic can be an ointment, a cream, a lotion, a spray, a powder, or a patch. The cosmetic may contain the dispersion formulation of the present disclosure and dermatologically acceptable additives (e.g., excipients, carriers, etc.).

### [Examples]

### Example 1: Effect of Emulsifiers

### Preparation of Solid Particle Dispersion Solution

10 mg of fluorescein-labeled hyaluronic acid was dissolved in 2 mL of MilliQ water. 200 mg of various emulsifiers shown in Table 1 were dissolved in 4 mL of cyclohexane. The aqueous phase and the organic phase were added to a glass vial and stirred at 26,000 rpm for 2 minutes using a Polytron homogenizer. The obtained emulsion was flash-frozen in liquid nitrogen and freeze-dried for 24 hours.

The entire amount of the solid obtained after drying was redispersed in 5 mL of olive oil to obtain a solid particle dispersion liquid.

**[Table 1]**

| **Table 1: List of Emulsifiers** | | |
|---|---|---|
| **Product Name** | **Cosmetic Display Name** | **Manufacturer** |
| ER290 | Sucrose erucate ester | Mitsubishi Chemical |
| DAO-7S | Polyglyceryl-10 decaoleate | Sakamoto Yakuhin Kogyo |
| CRS-75 | Polyglyceryl-6 polyricinoleate | Sakamoto Yakuhin Kogyo |
| PR-15 | Polyglyceryl-6 polyricinoleate | Nikko Chemicals |
| IS-202P | Polyglyceryl-2 diisostearate | Sakamoto Yakuhin Kogyo |
| IS-204P | Polyglyceryl-2 tetraisostearate | Sakamoto Yakuhin Kogyo |
| GMO HP | Glyceryl monooleate | CRODA |
| HCO-10 | Polyoxyethylene hydrogenated castor oil | Nikko Chemicals |
| PO-5S | Polyglyceryl-10 pentaoleate | Sakamoto Yakuhin Kogyo |
| IS-1009P | Polyglyceryl-10 decaisostearate | Sakamoto Yakuhin Kogyo |
| 700P2-C | Glyceryl monocaprate | Taiyo Kagaku |

### Measurement of Particle Size Distribution

The solid particle dispersion liquid was diluted 100-fold with cyclohexane, and the particle size distribution and Z-average particle size at 20°C were measured using a Zetasizer Nano Pro.

### Yucatan Micropig Skin Penetration Test

Yucatan micropig skin (4 months old) was thawed, and subcutaneous tissue was removed using a scalpel. It was cut into a size of 2 cm x 2 cm and sandwiched between the donor phase and acceptor phase of a vertical Franz cell with the stratum corneum facing upward.

The receptor phase was filled with 5 mL of PBS solution (pH 7.4) at 32°C. 100 µL of the solid particle dispersion solution was administered to the donor phase and incubated at 32°C for 24 hours.

After 24 hours, the solid particle dispersion solution in the donor phase was wiped off with a Kimwipe, and the two outermost layers of the stratum corneum were removed by tape stripping. The remaining stratum corneum was peeled off by tape stripping, and every 5 sheets were immersed in 1 mL of solution liquid (PBS/methanol/acetonitrile = 2/1/1, v/v) and extracted by shaking at room temperature for 22 hours. The skin other than the stratum corneum was divided into 16 parts with a scalpel, and similarly extracted by shaking at room temperature for 22 hours using 1 mL of PBS solution.

The extract was diluted 10-fold with PBS solution, and the fluorescence intensity was measured with a microplate reader to quantify the amount of hyaluronic acid.

Table 2 shows the composition of the prepared solid particle dispersion formulations. Table 3 shows the Z-average value and polydispersity index of particles in the solid particle dispersion formulations prepared by mixing sucrose fatty acid ester (ER-290) and various emulsifiers. Hyalo-Oligo (trademark) was used as the hyaluronic acid.

**[Table 2]**

| **Table 2: Composition of Prepared Formulations** | | | | |
|---|---|---|---|---|
| **Sample** | **Hyaluronic acid (mg)** | **ER290 (mg)** | **Emulsifier (mg)** | **Olive oil (mL)** |
| 100/0 | 2 | 40 | 0 | 1 |
| 25/75 | 2 | 10 | 30 | 1 |
| 50/50 | 2 | 20 | 20 | 1 |
| 75/25 | 2 | 30 | 10 | 1 |
| 0/100 | 2 | 0 | 40 | 1 |

**[Table 3]**

| **Table 3: Z-average value and polydispersity index of obtained particles and dispersibility in oily base** | | | | |
|---|---|---|---|---|
| **Emulsifier Type** | **ER290/<br>Other Emulsifier Ratio** | **Z-average (nm)** | **Polydispersity Index<br>(PDI)** | **Dispersibility<br>in Oil** |
| - | 100/0 | 510 ± 79 | 0.506 - 0.712 | S |
| 1009P | 75/25 | 452 ± 43 | 0.569 - 0.729 | B |
| | 50/50 | 279 ± 11 | 0.449 - 0.658 | B |
| | 25/75 | 178 ± 1 | 0.250 - 0.266 | S |
| | 0/100 | 172 ± 1 | 0.115 - 0.217 | C |
| 202P | 75/25 | 370 ± 14 | 0.548 - 0.838 | B |
| | 50/50 | 216 ± 4 | 0.405 - 0.427 | S |
| | 25/75 | 131 ± 1 | 0.184 - 0.207 | S |
| | 0/100 | 593 ± 23 | 0.274 - 0.338 | C |
| 204P | 75/25 | 766 ± 106 | 0.533 - 0.769 | B |
| | 50/50 | 648 ± 72 | 0.563 - 0.748 | B |
| | 25/75 | 750 ± 113 | 0.521 - 0.696 | B |
| | 0/100 | 189 ± 7 | 0.584 - 1.000 | C |
| POSS | 75/25 | 616 ± 113 | 0.481 - 0.707 | B |
| | 50/50 | 392 ± 64 | 0.477 - 0.743 | B |
| | 25/75 | 165 ± 6 | 0.325 - 0.348 | S |
| | 0/100 | 119 ± 1 | 0.131 - 0.150 | B |
| DAO7S | 75/25 | 439 ± 62 | 0.471 - 0.747 | S |
| | 50/50 | 366 ± 47 | 0.486 - 0.722 | S |
| | 25/75 | 244 ± 5 | 0.401 - 0.564 | S |
| | 0/100 | Not measurable*1 | | B |
| PR15 | 75/25 | 943 ± 107 | 0.631 - 0.695 | A |
| | 50/50 | 426 ± 49 | 0.423 - 0.519 | S |
| | 25/75 | 216 ± 1 | 0.371 - 0.397 | S |
| | 0/100 | 169 ± 5 | 0.186 - 0.196 | A |
| CRS75 | 75/25 | 1025 ± 229 | 0.680 - 0.766 | A |
| | 50/50 | 221 ± 1 | 0.550 - 0.590 | S |
| | 25/75 | 174 ± 2 | 0.349 - 0.367 | S |
| | 0/100 | 188 ± 4 | 0.213 - 0.231 | A |
| GMO HP | 75/25 | 1849 ± 301 | 0.796 - 0.856 | B |
| | 50/50 | 2655 ± 480 | 0.810 - 0.923 | B |
| | 25/75 | 2945 ± 393 | 0.854 - 0.947 | B |
| | 0/100 | 1448 ± 108 | 0.410 - 0.468 | C |
| HCO-10 | 75/25 | 2361 ± 36 | 0.396 - 0.673 | B |
| | 50/50 | 205 ± 4 | 0.464 - 0.655 | S |
| | 25/75 | 173 ± 2 | 0.255 - 0.264 | S |
| | 0/100 | 1288 ± 87 | 0.598 - 0.816 | B |

| | | | | |
|---|---|---|---|---|
| * In the table, symbol "S" represents being dispersed transparently or translucently, symbol "B" represents that precipitation has occurred, symbol "A" represents that there is no precipitation but it is cloudy, and symbol "C" represents that gelation or other inconveniences have occurred. Evaluation was performed by visual confirmation. *1 Example where numerical measurement was not possible because most precipitated and there were few dispersed particles. However, dispersed particles exist, and these particles penetrate the skin. | | | | |

Even in examples where the emulsifier alone resulted in precipitation of the transparent dispersion liquid, mixing two types of emulsifiers made it possible to prepare particles with a small Z-average value and a low polydispersity index. In many systems, the smallest Z-average value was shown when ER-290 was included at 25% of the total emulsifier. Also, emulsifiers with particularly high stabilizing effects were those with large and bulky head groups and tail groups, such as polyglyceryl-6 polyricinoleate and polyglyceryl-2 diisostearate. Emulsifiers where both are small, such as glyceryl monocaprate, tended to have a low stabilizing effect. On the other hand, with decaglycerin fatty acid esters, the hydrophilicity of the emulsifier increased, so the effect of stably dispersing the hydrophilic drug solid weakened. Similarly, the stabilizing effect was low with polyoxyethylene (10) hydrogenated castor oil. From this, it was suggested that the highest stabilizing effect may be obtained in the range where the polymerization number of glycerin units of the head group of the emulsifier is 2-6. In Tables 3 and 4, S is most preferable, A is preferable next to S, B is preferable next to A, and C is preferable next to B. Note that the presence of precipitation or cloudiness does not mean that there is no dispersion of solid particles in the liquid. Therefore, it should be noted that even if precipitation or cloudiness exists, penetration of solid particles into the skin cannot be ruled out. Therefore, even samples that produced cloudiness, precipitation, or gelation can be used for skin penetration applications.

In addition, long-term dispersibility was evaluated. After preparing the above solid particle dispersion formulation, the dispersibility of the formulation after storage at 25°C for 2 months was visually confirmed. The results were as shown in Table 4.

**[Table 4]**

| **Table 4: Long-term dispersibility test** | | |
|---|---|---|
| **Emulsifier Type ER290/Emulsifier Ratio Dispersibility after 2 months** | | |
| ER290 | 100/0 | B |
| 1009P | 75/25 | B |
| | 50/50 | B |
| | 25/75 | S |
| | 0/100 | B |
| 202P | 75/25 | B |
| | 50/50 | S |
| | 25/75 | S |
| | 0/100 | B |
| PO5S | 75/25 | B |
| | 50/50 | B |
| | 25/75 | S |
| | 0/100 | B |
| DOA7S | 75/25 | B |
| | 50/50 | S |
| | 25/75 | S |
| | 0/100 | B |
| PR15 | 75/25 | A |
| | 50/50 | S |
| | 25/75 | S |
| | 0/100 | B |
| CRS75 | 75/25 | A |
| | 50/50 | S |
| | 25/75 | S |
| | 0/100 | B |

| | | |
|---|---|---|
| * In the table, symbol "S" represents being dispersed transparently or translucently, symbol "B" represents that precipitation has occurred, and symbol "A" represents that there is no precipitation but it is cloudy. | | |

As shown in Table 4, when two emulsifiers were mixed, the long-term dispersibility of the formulation improved in all cases. Those skilled in the art would be able to appropriately adjust the mixing ratio of emulsifiers to obtain dispersibility or long-term dispersibility.

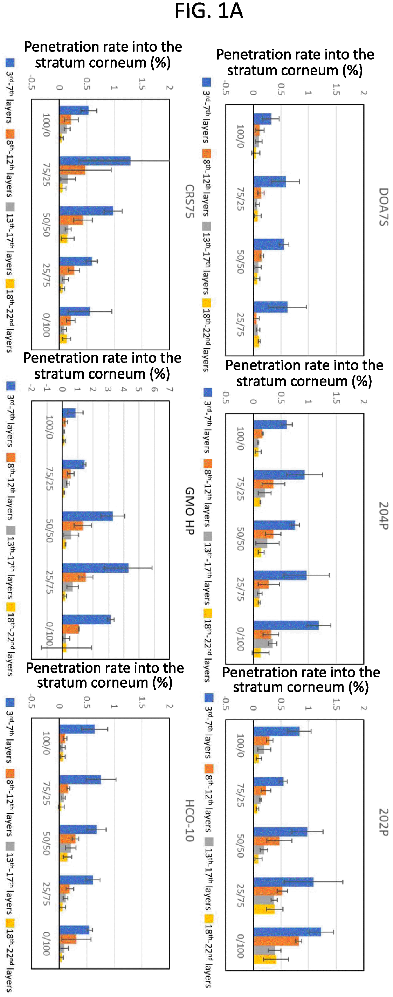

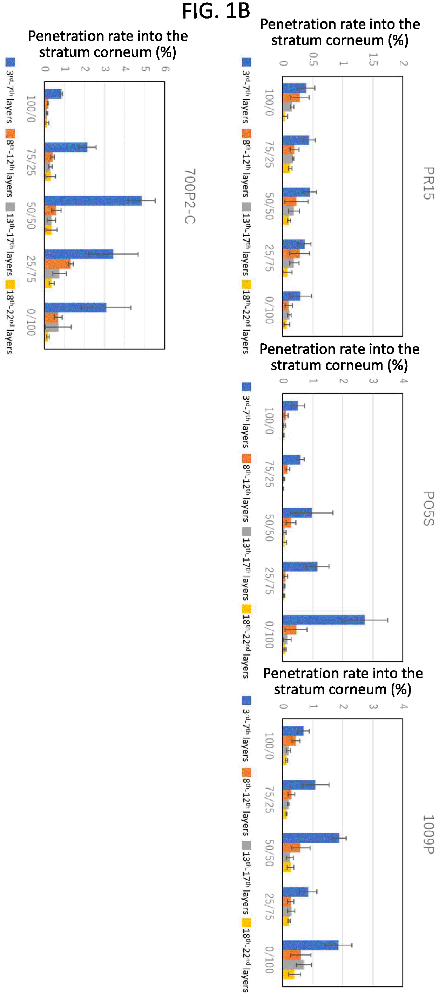

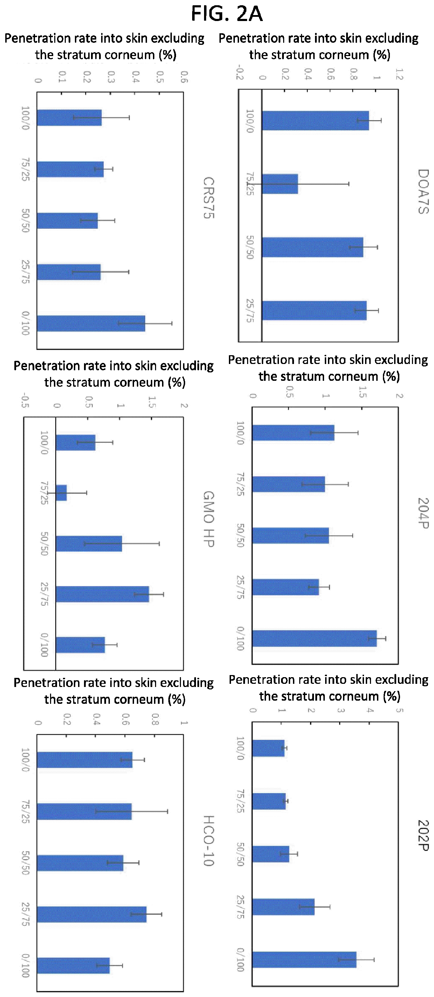

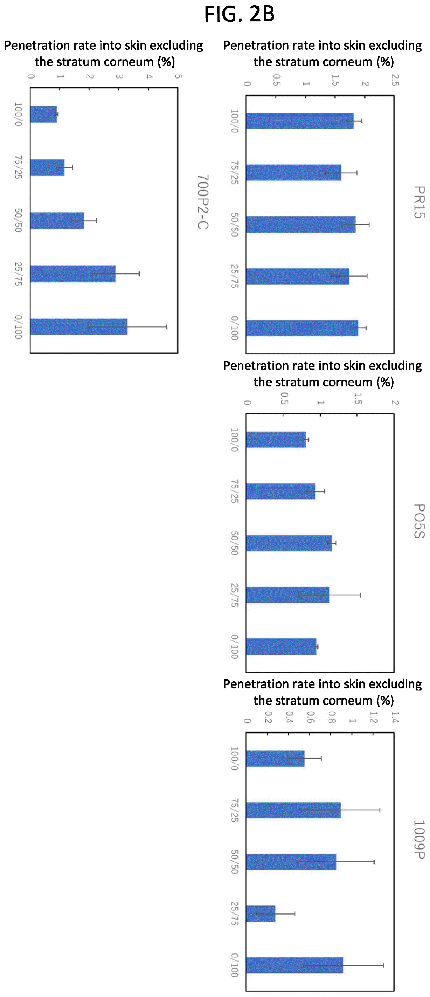

Figure 1 shows the amount of penetration into the skin when solid particle dispersion formulations prepared by mixing sucrose fatty acid ester (ER-290) and various emulsifiers were administered to Yucatan micropig skin. The Z-average value correlated with the permeability below the stratum corneum, and it became clear that the drug penetrated even to the layers below the stratum corneum in formulations with lower Z-average values. Furthermore, permeability to the hydrophilic region of the skin below the stratum corneum was also high (Fig. 2). The stratum corneum is in a state where keratin-filled corneocytes are paved within a hydrophobic region composed of ceramide double membranes, as likened to bricks and mortar. Therefore, it is considered that smaller particles were able to penetrate stably between corneocytes within the highly hydrophobic stratum corneum.

### Example 2: Solid Particle Dispersion Formulation of Extracellular Vesicles

ER-290 was obtained from Mitsubishi Chemical Corporation, and SY Glyster CRS-75 and S Face IS-202P were obtained from Sakamoto Yakuhin Kogyo Co., Ltd. Milk exosomes (Cosmo Bio) were stained with CoraLite594-labeled CD9 antibody (Proteintech Japan), and unreacted antibodies were removed using a PURE-EV column (HansaBioMed).

The obtained exosomes were mixed with a mixture of ER290 and CRS-75, or a mixture of ER290 and 202P in cyclohexane. By this operation, it is considered that particles having a core-shell structure containing exosomes in the core and emulsifier in the shell are formed. Cyclohexane was removed by freeze-drying to obtain solid complex particles having a core-shell structure. The solid complex particles were dispersed in IPM to prepare a solid particle dispersion formulation of exosomes.

The mixing amounts of each component used for the preparation of the solid particle dispersion formulation were as shown in Table 5.

**[Table 5]**

| **Table 5: Mixing amounts of each component used for the preparation of the solid particle dispersion formulation containing exosomes** | | | | |
|---|---|---|---|---|
| **Exosome / particles ER-290 (mg) CRS-75 (mg) IS-202P (mg) IPM (mL)** | | | | |
| Example 2-1 1.5 × 10⁸ | 10 | 10 | - | 1 |
| Example 2-2 1.5 × 10⁸ | 5 | - | 15 | 1 |

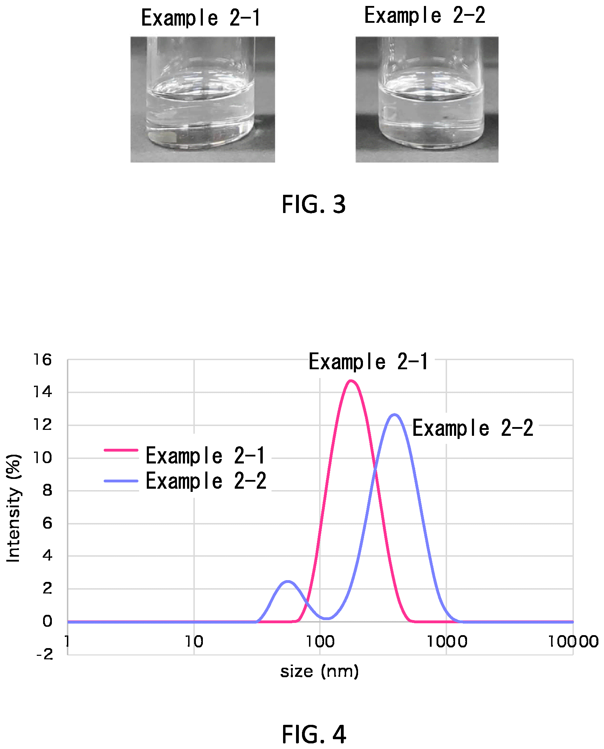

As shown in Fig. 3, the appearance of the obtained dispersion formulation was a transparent dispersion formulation. The particle size distribution of the obtained dispersion formulation at 25°C was measured using a Zetasizer Pro. The results were as shown in Fig. 4 and Table 6. As shown in Table 6, it became clear that a formulation having relatively small particles of 500 nm or less was obtained.

**[Table 6]**

| **Table 6: Particle size distribution of solid particle dispersion formulation containing exosomes** | | |
|---|---|---|
| peak1 | | PDI |
| Example 2-1 | 195 ± 2 | 0.1648 - 0.1948 |
| Example 2-2 | 415 ± 6 | 0.3596 - 0.4244 |

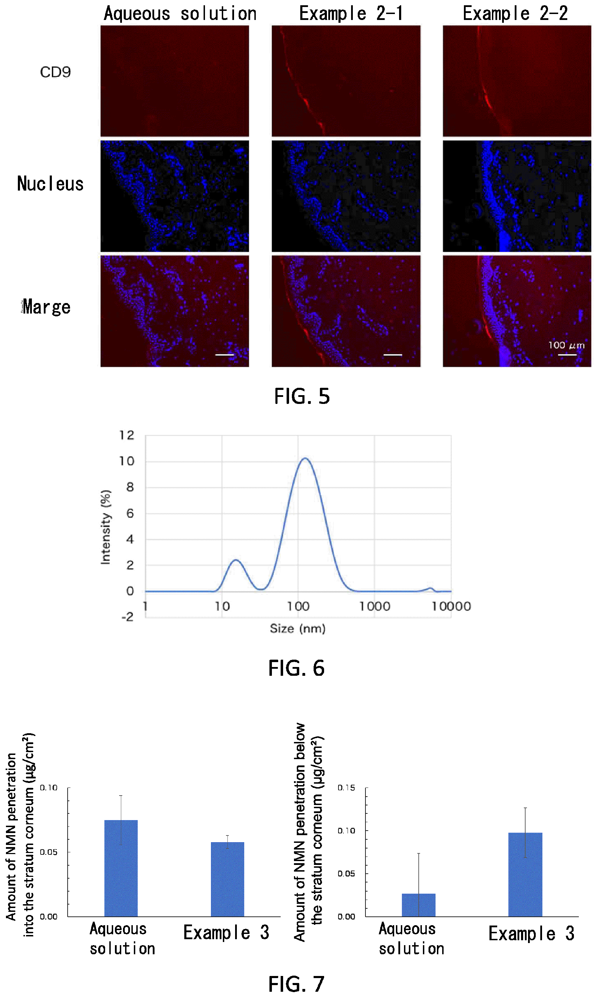

The obtained solid particle dispersion formulation containing exosomes was applied to the skin of micropigs, and its permeability was confirmed. Yucatan micropig skin (4 months old) was thawed, and subcutaneous tissue was removed using a scalpel. It was cut into a size of 2 cm x 2 cm and sandwiched between the donor phase and acceptor phase of a vertical Franz cell with the stratum corneum facing upward. The receptor phase was filled with 5 mL of PBS solution (pH 7.4) at 32°C. 100 µL of the dispersion formulation of Example 2-1 or 2-2 was administered to the donor phase and incubated at 32°C for 24 hours. After 24 hours, the dispersion formulation in the donor phase was wiped off with a Kimwipe, and the two outermost layers of the stratum corneum were removed by tape stripping. The remaining skin was frozen, and 8 µm frozen sections were prepared. The tissue sections of the skin were mounted with ProLong Glass Antifade Mountant with NucBlu Stain (Thermo Fisher Scientific), and the tissue was observed using a fluorescence microscope. The results were as shown in Fig. 5. As shown in Fig. 5, penetration of exosomes into the epidermis was observed by formulating into a dispersion formulation.

### Example 3: Aqueous Dispersion

4 mg of nicotinamide mononucleotide (NMN) was dissolved in 2 mL of MilliQ water. 19 mg of ER-290 and 57 mg of SY Glyster CRS-75 were dissolved in 4 mL of cyclohexane. The aqueous phase and the organic phase were placed in a glass vial and stirred at 26,000 rpm for 2 minutes using a Polytron homogenizer to form an emulsion. The obtained emulsion was flash-frozen in liquid nitrogen and freeze-dried for 24 hours.

20 mg of the liquid obtained after drying was dispersed in 1 mL of cyclohexane. 60 mg of NIKKOL BL-25 (Nikko Chemicals) was dissolved in 3 mL of water. The aqueous phase and the organic phase were placed in a glass vial and stirred at 26,000 rpm for 2 minutes using a Polytron homogenizer. The obtained emulsion was flash-frozen in liquid nitrogen and freeze-dried for 24 hours. By dispersing the liquid after freeze-drying in 1 mL of water, a dispersion liquid in which solid particles are dispersed in water (solid particle dispersion aqueous solution) was obtained (final NMN concentration: 1 mg/mL).

### Measurement of Particle Size Distribution

The obtained dispersion liquid was diluted 100-fold with water, and the particle size distribution at 25°C was measured using a Zetasizer Pro. The results were as shown in Fig. 6 and Table 7. As shown in Fig. 6 and Table 7, by applying the technology of the present disclosure to NMN, it was successfully made into solid particles and dispersed in an aqueous solution. The obtained particles had a particle size distribution of sub-micrometers.

**[Table 7]**

| **Table 7: Particle size distribution of solid particle dispersion aqueous solution** | | |
|---|---|---|
| peak1 | | PDI |
| Example 3 | 141 ± 9 | 0.450 - 0.460 |

### Yucatan Micropig Skin Penetration Test

Yucatan micropig skin (4 months old) was thawed, and subcutaneous tissue was removed using a scalpel. It was cut into a size of 2 cm x 2 cm and sandwiched between the donor phase and acceptor phase of a vertical Franz cell with the stratum corneum facing upward. The receptor phase was filled with 5 mL of PBS solution (pH 7.4) at 32°C. 150 µL of the obtained dispersion liquid was administered to the donor phase and incubated at 32°C for 24 hours. As a control, a 1 mg/mL NMN aqueous solution was used.

After 24 hours, the obtained dispersion liquid in the donor phase was wiped off with a Kimwipe, and the two outermost layers of the stratum corneum were removed by tape stripping. The remaining stratum corneum was peeled off by performing tape stripping 20 times, immersed in 5 mL of solution liquid (PBS/methanol/acetonitrile = 2/1/1, v/v), and extracted by shaking at room temperature for 22 hours. The skin other than the stratum corneum was divided into 16 parts with a scalpel, and extracted by shaking at room temperature for 22 hours using 1 mL of PBS solution.

The nuclear extract was diluted 10-fold with a diluent (0.1% formic acid/acetonitrile = 50/50, v/v), and the amount of NMN in the extract was quantified using LC-MS.

As shown in Fig. 7, the solid particle dispersion aqueous solution was able to allow a large amount of NMN to penetrate into the layers below the stratum corneum of the skin.

## Claims

1. A dispersion formulation, comprising solid particles containing a complex of a water-soluble molecule and one or more types of emulsifiers, and an oily base, wherein said one or more types of emulsifiers comprise a glyceryl fatty acid, and said solid particles are dispersed in said oily base.

2. The dispersion formulation according to claim 1, wherein the glyceryl fatty acid is selected from the group consisting of polyglyceryl polyfatty acid, polyglyceryl monofatty acid, monoglyceryl polyfatty acid, and monoglyceryl monofatty acid.

3. The dispersion formulation according to claim 1 or 2, wherein the glyceryl fatty acid comprises a polyglyceryl having a number average degree of polymerization of 2 to 6.

4. The dispersion formulation according to any one of claims 1 to 3, wherein said one or more types of emulsifiers further comprise a sucrose fatty acid ester.

5. The dispersion formulation according to any one of claims 1 to 4, wherein the sucrose fatty acid ester comprises a sucrose fatty acid ester.

6. The dispersion formulation according to any one of claims 1 to 5, wherein the sucrose fatty acid ester comprises sucrose erucate ester.

7. The dispersion formulation according to any one of claims 4 to 6, wherein said one or more types of emulsifiers comprise 10 to 40 mol% of the sucrose fatty acid ester.

8. The dispersion formulation according to any one of claims 1 to 7, wherein the Z-average size of the particles is 500 nm or less.

9. A cosmetic comprising the dispersion formulation according to any one of claims 1 to 8.

10. A dispersion formulation comprising solid particles containing a water-soluble molecule and one or more types of emulsifiers, and an aqueous base, wherein said solid particles are dispersed in said aqueous base.

11. The dispersion formulation according to claim 10, wherein said one or more types of emulsifiers comprise a sucrose fatty acid ester.

12. The dispersion formulation according to claim 10 or 11, wherein said one or more types of emulsifiers comprise sucrose erucate ester.

13. The dispersion formulation according to any one of claims 10 to 12, wherein the Z-average size of the particles is 200 nm or less.
